# EUROPÄISCHE PATENTSCHRIFT

(11) **EP 0 449 918 B1**
(45) Veröffentlichungstag und Bekanntmachung des Hinweises auf die Patenterteilung: **05.10.1994**
(21) Anmeldenummer: 90900856.7
(22) Anmeldetag: 15.12.1989
(51) Int. Cl.: C07C 69/54, C07C 67/08

(54) **VERFAHREN ZUR VERBESSERTEN HERSTELLUNG VON (METH)ACRYLSÄUREESTERN MEHRWERTIGER ALKOHOLE (I)**
IMPROVED PROCESS FOR PRODUCING ESTERS OF (METH)ACRYLIC ACID AND POLYHYDRIC ALCOHOLS (I)
PROCEDE AMELIORE DE PRODUCTION D'ESTERS D'ACIDE (METH)ACRYLIQUE D'ALCOOLS POLYVALENTS (I)

(30) Priorität: 24.12.1988 DE 3843854
(43) Veröffentlichungstag der Anmeldung: 09.10.1991
(73) Patentinhaber: Henkel Kommanditgesellschaft auf Aktien, 40191 Düsseldorf (DE)
(72) Erfinder: RITTER, Wolfgang, D-5657 Haan (DE); SITZ, Hans-Dieter, D-4049 Rommerskirchen (DE); SPEITKAMP, Ludwig, D-4000 Düsseldorf 13 (DE)
(74) Vertreter: von Kreisler, Alek, Dipl.-Chem.
(86) Internationale Anmeldenummer: EP8901549
(87) Internationale Veröffentlichungsnummer: WO9007486

(56) Entgegenhaltungen:
- DE-A- 2 552 987
- JP-A- 5 531 046
- US-A- 4 187 383

## Beschreibung

Die Erfindung betrifft ein Verfahren zur Herstellung von polyfunktionellen Estern der Acrylsäure und/oder der Methacrylsäure mit mehrwertigen Alkoholen - im folgenden auch als (Meth)acrylsäureester bzw. Poly(Meth)acrylsäureester bezeichnet - durch Umsetzung der Reaktanten in Gegenwart saurer Veresterungskatalysatoren unter Zusatz von Polymerisationsinhibitoren zum Reaktionsgemisch.

(Meth)acrylsäureester mehrwertiger Alkohole insbesondere aus der Gruppe der 2- bis 4wertigen aliphatischen gesättigten Alkohole und deren Oxalkylierungsprodukte finden zunehmende Bedeutung als hochreaktive Bestandteile in strahlenhärtenden Systemen. Solche polyfunktionellen (Meth)acrylsäureester können beispielsweise als Lackrohstoffe für die Elektronenstrahlhärtung oder als Bestandteil von UV-Licht-härtenden Druckfarben oder entsprechenden Überzugslacken, Spachtel, Form- oder Vergußmassen sowie in Klebstoffen, insbesondere anaerob härtenden Klebstoffen Verwendung finden. Ihre Herstellung ist allerdings nicht problemlos. Gefordert werden insbesondere farblose Produkte mit geringer Säurezahl und hoher Lagerstabilität, die praktisch auch keinen Eigengeruch aufweisen. Eine destillative Reinigung der (Meth)acrylsäureester der hier betroffenen Art scheidet in aller Regel aufgrund ihres hohen Molekulargewichtes und ihrer hohen Reaktivität aus. Die Produkte sollen also unmittelbar als möglichst farblose Reaktionsprodukte der Veresterung anfallen. Die Durchführung der Veresterungsreaktion fordert die Mitverwendung hochwirksamer Inhibitoren, die ihrerseits keine unerwünschten Nebenreaktionen, beispielsweise Verfärbungen, auslösen.

Zur Herstellung solcher polyfunktioneller (Meth)acrylsäureester mehrwertiger Alkohole besteht umfangreiche Vorliteratur. Verwiesen sei insbesondere auf die deutsche Offenlegungsschrift 29 13 218 und die darin zitierte einschlägige Literatur. So ist es aus der DE-AS 12 67 547 und aus der Zeitschrift "Chem. and Ind." 18 (1970), 597, bekannt, polyfunktionelle (Meth)acrylsäureester durch azeotrope Veresterung der (Meth)acrylsäure mit mehrwertigen Alkoholen in Gegenwart von azeotropen Schleppmitteln sowie von sauren Katalysatoren und Polymerisationsinhibitoren herzustellen. Als Polymerisationsinhibitoren wurden vorgeschlagen Phenole, Phenolderivate, Kupfer, Kupferverbindungen oder Phenothiazin. Als saure Katalysatoren werden organische oder anorganische Säuren oder saure Ionenaustauscher eingesetzt, wobei p-Toluolsulfonsäure und Schwefelsäure bevorzugt sein können. Die Veresterung erfolgt insbesondere bei Temperaturen von 40 bis 120 °C. Geeignete azeotrope Schleppmittel für die Entfernung des Reaktionswassers sind aliphatische oder cycloaliphatische oder aromatische Kohlenwasserstoffe bzw. deren Gemische mit Siedebereichen innerhalb der angegebenen Temperaturgrenzen.

In der genannten DE-OS 29 13 218 wird vorgeschlagen, die azeotrope Veresterung in Gegenwart mindestens eines organischen Esters der phosphorigen Säure zusätzlich zu einem mitverwendeten Inhibitor auf Phenolbasis durchzuführen. Zwingend wird allerdings auch hier die Mitverwendung mindestens eines aliphatischen, cycloaliphatischen und/oder aromatischen Kohlenwasserstoffs mit einem Siedepunkt im Bereich von 40 bis 120 °C gefordert. Das entstehende Reaktionswasser soll mittels dieser Schleppmittel azeotrop ausgekreist werden. Die Reaktionsdauer wird nach den Beispielen dieser Druckschrift mit 10 bis 18 Stunden angesetzt.

Die Erfindung geht von der Aufgabe aus, Reaktionsbedingungen für die hier betroffene Veresterungsreaktion zu ermitteln, die einerseits zu einer substantiellen Abkürzung der Reaktionsdauer führen können, andererseits aber die Qualität der entstehenden Veresterungsprodukte und insbesondere die hohe Farbqualität nicht negativ beeinflussen. Die Erfindung will weiterhin darauf verzichten können, vergleichsweise komplexe Inhibitorsysteme derart einsetzen zu müssen, wie sie in der genannten DE-OS 29 13 218 beschrieben sind. Es soll dabei erfindungsgemäß auch möglich sein, den im praktischen Einsatz gewünschten Applikationsinhibitor der hier betroffenen hochreaktiven Systeme gleichzeitig schon als Reaktionsinhibitor bei der Synthese der polyfunktionellen (Meth)acrylsäureester einzusetzen.

Die technische Lösung der erfindungsgemäßen Aufgabenstellungen geht von der Erkenntnis aus, daß vergleichsweise hochreine Veresterungsprodukte als unmittelbare Verfahrensprodukte selbst dann erhalten werden können, wenn auf die Mitverwendung von Verdünnungsmitteln bzw. azeotropen Schleppmitteln verzichtet wird und daß es unter den Bedingungen des lösungsmittelfreien Arbeitens sogar möglich ist, vergleichsweise schärfere Veresterungsbedingungen einzusetzen, die zu einer beträchtlichen Abkürzung der Reaktionszeit führen. Voraussetzung hierfür ist insbesondere, daß der richtige Polymerisationsinhibitor gewählt und unter den nachfolgenden geschilderten Verfahrensbedingungen gearbeitet wird.

Gegenstand der Erfindung ist dementsprechend ein Verfahren zur Herstellung von (Meth)acrylsäureestern mehrwertiger Alkohole durch deren Umsetzung mit Acrylsäure und/oder Methacrylsäure in Gegenwart von sauren Veresterungskatalysatoren unter Zusatz von alpha-substituierten phenolischen Verbindungen als Polymerisationsinhibitoren. Das neue Verfahren ist dadurch gekennzeichnet, daß man mit bei Reaktionstemperatur flüssigen Reaktionsgemischen ohne Zusatz Von Lösungs- und/oder azeotropen Schleppmitteln bei einer Sumpftemperatur von wenigstens 90 °C arbeitet, den Reaktionsraum mit einem Gasstrom durchspült und dabei das entstehende Kondensationswasser aus der Gasphase des Reaktionsraumes abzieht. Als bevorzugter Polymerisationsinhibitor aus der Klasse der alpha-substituierten phenolischen Verbindungen wird das 2,5-Di-tert.-butylhydrochinon eingesetzt.

Zur Ausschleusung des Kondensationswassers aus der Veresterungsreaktion aus dem Reaktor wird bevorzugt mit einem Gasstrom gearbeitet, der einen beschränkten Anteil an freiem Sauerstoff enthält. In Abhängigkeit von den jeweils gewählten Verfahrensbedingungen kann Luft oder ein an Sauerstoff verarmtes Gasgemisch eingesetzt werden. Ein Beispiel der zuletzt genannten Art sind Stickstoff/Luft-Gemische. In aller Regel wird allerdings ein gewisser Gehalt an freiem Sauerstoff in dieser der Reaktionsmischung zugeführten Gasphase gewünscht. Diese beschränkten Sauerstoffmengen aktivieren in an sich bekannter Weise den Inhibitor während des Reaktionsgeschehens.

Der Sauerstoffgehalt des Gasgemisches liegt im allgemeinen bei wenigstens etwa 1 Volumen-% und bevorzugt im Bereich von etwa 2 bis 20 Volumen-%. Aus Gründen der Reaktionssicherheit kann es dabei bevorzugt sein, mit Gehalten an freiem Sauerstoff in der unteren Hälfte des genannten Bereiches, also bis etwa 10 Volumen-% und bevorzugt bis etwa 7 Volumen-% zu arbeiten. Der Gasstrom wird in einer bevorzugten Ausführungsform der Erfindung in das flüssige Reaktionsgemisch eingespeist und kann dieses beispielsweise feinverteilt durchperlen. Es wird dabei zweckmäßigerweise mit begrenzten Mengen dieses Gasstromes gearbeitet, so daß kein unerwünscht hoher Austrag an Reaktionskomponenten - insbesondere der vergleichsweise leichter flüchtigen Säuren - stattfindet.

Als Polymerisationsinhibitoren können bestimmt ausgewählte einzelne Verbindungen als auch mehrere Komponenten aus der Klasse der alpha-substituierten Phenolverbindungen eingesetzt werden. Bevorzugt sind vergleichsweise schwer flüchtige Verbindungen, insbesondere auf Basis entsprechend substituierter einwertiger oder mehrwertiger Phenole, wobei als mehrwertige Phenolverbindungen insbesondere zweiwertige Phenoltypen von der Art der di-substituierten Hydrochinonderivate in Betracht kommen. Weitere Beispiele sind p-Methoxiphenol, 2,5-Di-tert-butyl-p-cresol, Methylhydrochinon und/oder tert.-Butylbrenzkatechin. Der bevorzugte Inhibitor ist das bereits genannte 2,5-Di-tert.-butylhydrochinon. Insbesondere mit diesem Inhibitor gelingt die Herstellung von strahlenhärtbaren polyfunktionellen (Meth)acrylsäureestern mit hoher Reinheit und insbesondere geringer Eigenfarbe auch unter den vergleichsweise scharfen erfindungsgemäß gewählten Verfahrensbedingungen. Die dabei problemlos anfallenden polyfunktionellen (Meth)acrylsäureester zeichnen sich zudem durch hohe Lagerstabilität aus.

Der Polymerisationsinhibitor bzw. gegebenenfalls das Inhibitorgemisch wird dem Reaktionsgemisch üblicherweise in Mengen von 200 bis 10000 ppm und bevorzugt im Bereich von etwa 300 bis 2000 ppm zugesetzt. Die Zahlenangaben beziehen sich dabei jeweils auf das Gewicht des aus (Meth)acrylsäure und polyfunktionellen Alkoholen bestehenden Reaktionsgemisches.

Als zu veresternde Polyalkohole seien beispielsweise genannt: Ethylenglycol, Propylenglycol, Butandiol-1,4, Hexandiol-1,6, Neopentylglycol, Diethylenglycol, Triethylenglycol, Dimethylolpropan, Glycerin, Trimethylolpropan, Trimethylolhexan, Trimethylolethan, Hexantriol-1,3,5 und Pentraerythrit. Erfindungsgemäß kommen als polyfunktionelle Alkohole insbesondere aber auch die Oxalkylierungsprodukte dieser zuvor genannten polyfunktionellen Alkohole in Betracht, wobei hier den Oxethylierungsprodukten und/oder den Oxpropylierungsprodukten besondere Bedeutung zukommt. Kettenverlängerte polyfunktionelle Alkohole dieser Art können beträchtliche Mengen an Polyalkoxidresten enthalten, beispielsweise 1 bis 50 Mol, vorzugsweise etwa 1 bis 20 Mol Ethylenoxid pro g-Äquivalent an Hydroxylgruppen.

Veresterungskatalysatoren für das erfindungsgemäße Herstellungsverfahren sind handelsübliche organische oder anorganische Säuren oder auch saure Ionenaustauscher, wobei den in der Praxis häufig eingesetzten entsprechenden Verbindungen p-Toluolsulfonsäure und Schwefelsäure besondere Bedeutung zukommt. Die Mengen des Veresterungskatalysators liegen beispielsweise im Bereich von 0,1 bis 5 Gew.-% bezogen auf das Veresterungsgemisch.

Die Umsetzung der Reaktanten wird vorzugsweise bei Sumpftemperaturen von wenigstens etwa 100 °C durchgeführt. Besonders geeignet ist der Temperaturbereich bis etwa 150 °C. Dabei kann unter Normaldruck, zweckmäßigerweise aber auch unter abgesenktem Druck gearbeitet werden. Beim Arbeiten mit vermindertem Druck kann in einer besonderen Ausführungsform der Druck in Richtung auf niedrigere Drucke stufenweise oder auch kontinuierlich verringert werden.

Durch die Möglichkeit unter vergleichsweise scharfen Veresterungsbedingungen und gleichzeitig vermindertem Druck zu arbeiten, wird die Reaktionsdauer gegenüber bisher beschriebenen Verfahren stark abgekürzt. So können im erfindungsgemäßen Verfahren Umsatzausbeuten von wenigstens 90 % der Theorie und vorzugsweise von wenigstens etwa 94 % der Theorie im Temperaturbereich von etwa 100 bis 140 °C bei einer Reaktionsdauer von nicht mehr als etwa 10 Stunden und vorzugsweise von nicht mehr als etwa 8 Stunden erzielt werden. Gleichwohl fallen die Reaktionsprodukte als hellfarbige oder durch eine einfache Nachbehandlung wirkungsvoll zu reinigende stabilisierte Masse an. Das den sauren Veresterungskatalysator enthaltende Reaktionsrohprodukt wird einer nachfolgenden Neutralisation unterworfen. Diese Neutralisation kann unter bekannten Naßbedingungen, beispielsweise durch Einsatz von wäßrigen Soda- und gegebenenfalls Natriumchlorid enthaltenden Lösungen erfolgen. In einer bevorzugten Ausführungsform wird allerdings das den sauren Katalysator enthaltende Reaktionsrohprodukt einer trockenen Neutralisation unterworfen. Geeignete trockene Neutralisationsmittel sind die Oxide und/oder Hydroxide der Alkalimetalle, der Erdalkalimetalle und/oder des Aluminiums. Besonders geeignet sind zur Trockenneutralisation entsprechende Verbindungen des Magnesiums bzw. des Calciums.

(Meth)acrylsäure und die Alkohole können für die Veresterung in äquivalenten Mengenverhältnissen eingesetzt werden. Bei den mehr als zweiwertigen Alkoholen ist es allerdings auch ohne weiteres möglich, nur einen Teil der Hydroxylgruppen zu verestern. Zur Vollveresterung kann es zweckmäßig sein, die Säurekomponente in leichtem Überschuß über die zur Veresterung der Hydroxylgruppen erforderliche stöchiometrische Menge einzusetzen. Ein solcher Überschuß kann wenigstens etwa 10 Mol-% ausmachen. Nach Abschluß der Reaktion kann gewünschtenfalls zusätzlich ein Inhibitor dem Reaktionsprodukt beigemischt werden.

Treten bei der Herstellung der Reaktionsprodukte unter den erfindungsgemäßen drastischen Veresterungsbedingungen dann doch einmal leichte Farbverschlechterungen im Reaktionsprodukt auf, so lassen sich diese problemlos durch eine Nachbehandlung mit Entfärbungsmitteln beseitigen. Ein geeignetes Entfärbungsmittel ist beispielsweise Aluminiumoxid.

### Beispiele

### Beispiel 1

In einem 3-Liter-Reaktor wurden 1559,5 g Acrylsäure, 1521,0 g eines ethoxylierten Trimethylolpropans (OH-Zahl: 680 mg KOH/g Substanz), 107,8 g p-Toluolsulfonsäure sowie 4,96 g 2,5-Di-tert.-butylhydrochinons (2000 ppm bezogen auf Produktmenge) eingewogen.

Die Veresterung wurde unter Durchleiten von Luft (40 l/h) und unter Wasserabtrennung durchgeführt. Bei einer maximalen Sumpftemperatur von 105 °C und einem Druck von 400 mbar betrug die Veresterungszeit 6 Stunden.

| Rohprodukt: | |
|---|---|
| Säurezahl: | 31 mg KOH/g |
| OH-Zahl: | 21,8 mg KOH/g |
| Gardner Farbzahl: | 94,4 % |
| H₂O-Gehalt: | 0,15 % |

Das Rohprodukt wurde durch Zugabe von 103 g Ca(OH)₂ und 2-stündigem Rühren bei 80 °C und 50 mbar neutralisiert und anschließend mit Hilfe einer Druckfilternutsche filtriert.

| Produkt: | |
|---|---|
| Säurezahl: | < 1 mg KOH/g |
| OH-Zahl: | 24 mg KOH/g |
| Gardner Farbzahl: | < 1 |

### Beispiel 2

Beispiel 1 wurde mit der Änderung wiederholt, daß die Veresterung bei 120 °C und 700 mbar durchgeführt wurde.

| Rohprodukt: | |
|---|---|
| Säurezahl: | 20 mg KOH/g |
| OH-Zahl: | 20 mg KOH/g |
| Umsatz: | 95,0 % |
| Gardner Farbzahl: | 3 |

Das Rohprodukt wurde mit 4 Liter wäßriger 16 Gew.-% NaCl/4 Gew.-% NaHCO₃-Lösung gewaschen, mit 200 ppm Hydrochinonmonomethylether nachinhibiert und im Vakuum bei 40 mbar und 80 °C 2 Stunden getrocknet und mit Hilfe einer Druckfilternutsche filtriert.

| Produkt: | |
|---|---|
| Säurezahl: | < 1 mg KOH/g |
| OH-Zahl: | 23 mg KOH/g |
| Gardner Farbzahl: | 2 - 3 |

### Beispiel 3

Die Veresterung wurde wie in Beispiel 1 durchgeführt mit der Änderung, daß die Veresterung bei 140 °C und Normaldruck durchgeführt wurde.

| Rohprodukt: | |
|---|---|
| Säurezahl: | 64 mg KOH/g |
| OH-Zahl: | 29 mg KOH/g |
| Umsatz: | 92,5 % |
| Gardner Farbzahl: | 4 |

Das Rohprodukt wurde analog zu Beispiel 2 aufgearbeitet.

| Produkt: | |
|---|---|
| Säurezahl: | < 1 mg KOH/g |
| OH-Zahl: | 33 mg KOH/g |
| Gardner Farbzahl: | 3 - 4 |

### Beispiel 4

1320,0 g Acrylsäure, 1861,7 g eines propoxylierten Neopentylglycols (OH-Zahl: 460 mg KOH/g Substanz) sowie 111,4 g p-Toluolsulfonsäure wurden in einen 3-Liter-Reaktor eingewogen und mit 5,37 g 2,5-Di-tert.-butylhydrochinon (2000 ppm bezogen auf Produktmenge) inhibiert. Unter Durchleiten von Luft (40 l/h) wurde die Veresterung unter Wasserabtrennung durchgeführt. Bei einer maximalen Sumpftemperatur von 105 °C bei 400 mbar betrug die Veresterungszeit 6 Stunden.

| Rohprodukt: | |
|---|---|
| Säurezahl: | 34 mg KOH/g |
| OH-Zahl: | 17 mg KOH/g |
| Umsatz: | 94,4 % |
| Gardner Farbzahl: | < 1 |

Das Rohprodukt wurde durch Zugabe von 125 g Ca(OH)₂ und 2-stündigem Rühren bei 80 °C und 50 mbar neutralisiert und anschließend mit Hilfe einer Druckfilternutsche filtriert.

| Produkt: | |
|---|---|
| Säurezahl: | < 1 mg KOH/g |
| OH-Zahl: | 20 mg KOH/g |
| Gardner Farbzahl: | < 1 |

### Beispiel 5

368,2 g eines ethoxylierten Trimethylolpropans (OH-Zahl 680 mg KOH/g Substanz), 376,2 g Acrylsäure, 26,5 g p-Toluolsulfonsäure und 0,18 g 2,5-Di-tert.-butylhydrochinon (300 ppm bezogen auf die Produktmenge) wurden in einen 1-Liter-Dreihalskolben eingewogen.

Unter Durchleiten von Luft (20 l/h) wurde die Veresterung unter Wasserabscheidung durchgeführt. Bei einer maximalen Reaktionstemperatur von 105 °C und einem Vakuumsprofil von 2 h/400 mbar, 1 h/270 mbar, 1 h/200 mbar und 1 h/50 mbar betrug die Veresterungszeit 5 Stunden.

| Rohprodukt: | |
|---|---|
| Säurezahl: | 39,2 mg KOH/g |
| OH-Zahl: | 18 mg KOH/g |
| Umsatz: | 95,4 % |
| Gardner Farbzahl: | 2 |
| H₂O-Gehalt: | 0,13 % |

Das Rohprodukt wurde durch Zugabe von 31,8 g Ca(OH)₂ und 2stündigem Rühren bei 80 °C und 50 mbar neutralisiert und anschließend mit Hilfe einer Druckfilternutsche filtriert.

| Produkt: | |
|---|---|
| Säurezahl: | < 1 mg KOH/g |
| OH-Zahl: | 21 mg KOH/g |
| Gardner Farbzahl: | < 1 |
| H₂O-Gehalt: | 0,17 % |

## Patentansprüche

1. Verfahren zur Herstellung von (Meth)acrylsäureestern mehrwertiger Alkohole durch Umsetzung der Reaktanten in Gegenwart von sauren Veresterungskatalysatoren unter Zusatz von alpha-substituierten phenolischen Verbindungen, insbesondere Di-tert.-butylhydrochinon, als Polymerisationsinhibitoren, dadurch gekennzeichnet, daß man ohne Zusatz von Lösungs- und/oder azeotropen Schleppmitteln bei einer Sumpftemperatur von wenigstens 90 °C mit bei Reaktionstemperatur flüssigen Reaktionsgemischen arbeitet, den Reaktionsraum mit einem Gasstrom durchspült und dabei das entstehende Kondensationswasser aus der Gasphase des Reaktionsraumes abzieht.

2. Verfahren nach Anspruch 1, dadurch gekennzeichnet, daß man den Reaktionsraum mit einem freien Sauerstoff enthaltenden Gasstrom durchspült.

3. Verfahren nach Ansprüchen 1 und 2, dadurch gekennzeichnet, daß man als Gasstrom Luft oder O₂-abgereicherte Gasgemische insbesondere Stickstoff/Luftgemische einsetzt.

4. Verfahren nach Ansprüchen 1 bis 3, dadurch gekennzeichnet, daß die Veresterung bei Sumpftemperaturen von wenigstens 100°C und insbesondere im Bereich bis etwa 150 °C durchgeführt wird, wobei bevorzugt wenigstens Zeitweise bei vermindertem, gegebenenfalls stufenweise zunehmend vermindertem Druck, gearbeitet wird.

5. Verfahren nach Ansprüchen 1 bis 4, dadurch gekennzeichnet, daß die Inhibitoren in Mengen von 200 bis 10000 ppm, bevorzugt im Bereich von 300 bis 2000 ppm - jeweils bezogen auf das Gewicht des Reaktionsgemisches - eingesetzt werden,

6. Verfahren nach Ansprüchen 1 bis 5, dadurch gekennzeichnet, daß man im Temperaturbereich von 100 bis 140 °C - gewünschtenfalls unter Vakuum - für eine Reaktionsdauer von nicht mehr als 10 Stunden, insbesondere von nicht mehr als 8 Stunden, arbeitet und dabei die Reaktion auf einen Umsatz von wenigstens 90 % der Theorie, vorzugsweise von wenigstens 94 % der Theorie führt.

7. Verfahren nach Ansprüchen 1 bis 6, dadurch gekennzeichnet, daß das Reaktionsrohprodukt einer trockenen Neutralisation - bevorzugt mit Oxiden und/oder Hydroxiden der Alkalimetalle, der Erdalkalimetalle und/oder des Aluminium - unterworfen wird.

8. Verfahren nach Ansprüchen 1 bis 7, dadurch gekennzeichnet, daß die primär anfallenden Reaktionsprodukte einer abschließenden Behandlung mit Entfärbungsmitteln unterworfen werden.

## Claims

1. A process for the production of (meth)acrylic acid esters of polyhydric alcohols by reaction of the reactants in the presence of acidic esterification catalysts with addition of α-substituted phenolic compounds, more particularly ditert.butyl hydroquinone, as polymerization inhibitors, characterized in that the process is carried out in the absence of solvents and/or azeotropic entraining agents at a sump temperature of at least 90°C using reaction mixtures which are liquid at room temperature, a gas stream is passed through the reaction zone and the water of condensation formed is removed from the gas phase of the reaction zone.

2. A process as claimed in claim 1, characterized in that the reaction zone is purged with a gas stream containing free oxygen.

3. A process as claimed in claims 1 and 2, characterized in that air or O₂-depleted gas mixtures, more particularly nitrogen/air mixtures, are used as the gas stream.

4. A process as claimed in claims 1 to 3, characterized in that the esterification reaction is carried out at sump temperatures of at least 100°C and, more preferably, in the range up to about 150°C, the reaction preferably being carried out at least periodically under reduced pressure and optionally under a pressure increasingly reduced in steps.

5. A process as claimed in claims 1 to 4, characterized in that the inhibitors are used in quantities of from 200 to 10,000 ppm and preferably in quantities of from 300 to 2,000 ppm, based on the weight of the reaction mixture.

6. A process as claimed in claims 1 to 5, characterized in that the reaction is carried out at a temperature in the range from about 100 to 140°C, optionally in vacuo, over a reaction time of no more than 10 hours and, in particular, no more than 8 hours and is continued to a yield of at least 90% of the theoretical and preferably of at least 94% of the theoretical.

7. A process as claimed in claims 1 to 6, characterized in that the crude reaction product is subjected to dry neutralization, preferably with oxides and/or hydroxides of the alkali metals, the alkaline earth metals and/or aluminium.

8. A process as claimed in claims 1 to 7, characterized in that the reaction products initially obtained are subjected to a final treatment with decolouring agents.

## Revendications

1. Procédé de production d'esters d'acide (méth)acrylique d'alcools polyvalents par conversion des réactants en présence de catalyseurs d'estérification acides en ajoutant des composés phénoliques alpha-substitués, en particulier de la di-tert-butyl-hydroquinone, comme inhibiteurs de polymérisation, caractérisé en ce qu'on opère sans ajouter de solvants et/ou d'agents entraînants azéotropes à une température de distillation d'au moins 90°C avec des mélanges réactionnels liquides à la température de réaction, qu'on fait traverser par barbotage l'espace réactionnel par un courant gazeux et qu'on retire en outre de la phase gazeuse de l'espace réactionnel l'eau de condensation résultante.

2. Procédé selon la revendication 1, caractérisé en ce qu'on fait traverser par barbotage l'espace réactionnel par un courant gazeux contenant de l'acide libre.

3. Procédé selon les revendications 1 ou 2, caractérisé en ce qu'on utilise comme courant gazeux de l'air ou des mélanges gazeux appauvris en O₂ en particulier des mélanges air/azote.

4. Procédé selon les revendications 1 à 3, caractérisé en ce que l'estérification est réalisée à des températures de distillation d'au moins 100°C et en particulier dans la plage jusqu'à environ 150°C, tandis que de préférence on opère au moins par moments à une pression réduite, éventuellement réduite de plus en plus par étapes.

5. Procédé selon les revendications 1 à 4, caractérisé en ce que les inhibiteurs sont mis en oeuvre en quantités de 200 à 1000 ppm, de préférence dans la gamme de 300 à 2000 ppm respectivement par rapport au poids du mélange réactionnel.

6. Procédé selon les revendications 1 à 5, caractérisé en ce qu'on opère dans la plage de température de 100 à 140°C, éventuellement sous vide, pour une durée de réaction de pas plus de 10 heures, en particulier de pas plus de 8 heures et qu'en outre la réaction conduit à une conversion d'au moins 90 % de la théorie, de préférence d'au moins 94 % en théorie.

7. Procédé selon les revendications 1 à 6, caractérisé en ce que le produit brut de réaction est soumis à une neutralisation à sec, de préférence avec des oxydes et/ou des hydroxydes des métaux alcalins, des métaux alcalino-terreux et/ou de l'aluminium.

8. Procédé selon les revendications 1 à 7, caractérisé en ce que les produits de réaction résultant en premier lieu sont soumis à un traitement final avec des agents décolorants.
